# EUROPÄISCHE PATENTSCHRIFT

(11) **EP 1 123 916 B1**
(45) Veröffentlichungstag und Bekanntmachung des Hinweises auf die Patenterteilung: **04.08.2004**
(21) Anmeldenummer: 01101657.3
(22) Anmeldetag: 29.01.2001
(51) Int. Cl.: C07C 209/10, C07C 209/36, C07C 211/55

(54) **Verfahren zur Herstellung von Aminodiphenylaminen**
Process for the preparation of amino diphenylamines
Procédé pour la préparation d'amino-diphénylamines

(30) Priorität: 09.02.2000 DE 10005601
(43) Veröffentlichungstag der Anmeldung: 16.08.2001
(73) Patentinhaber: BAYER AG, 51368 Leverkusen (DE)
(72) Erfinder: Giera, Henry, Dr., 86862 Grosskitzighofen (DE); Pohl, Torsten, Dr., Cambridge, MA 02138 (US); Hugger, Uwe, Dr., 25462 Rellingen (DE); Sicheneder, Adolf, Dr., 25551 Hohenlockstedt (DE); Schumacher, Fred, 25560 Schenefeld (DE); Brill, Adolf, 25541 Brunsbüttel (DE)

(56) Entgegenhaltungen:
- EP-A- 1 081 128
- DE-A- 3 246 151
- US-A- 5 840 982
- CHRISTIAN S. RONDESTVEDT JR.: "Synthesis of 4-Aminodiphenylamine and Its Relatives" JOURNAL OF ORGANIC CHEMISTRY., Bd. 42, Nr. 10, 1977, Seiten 1786-1790, XP002166757 AMERICAN CHEMICAL SOCIETY. EASTON., US ISSN: 0022-3263

## Beschreibung

Die Erfindung betrifft ein Verfahren zur Herstellung von Aminodiphenylaminen, insbesondere 4-Aminodiphenylamin (4-ADPA), durch Umsetzung von Nitrohalogenbenzolen mit aromatischen Aminen in Gegenwart eines Palladium-Katalysators und einer Base und anschließender Hydrierung des so erhaltenen Zwischenproduktes.

4-Aminodiphenylamin (4-ADPA) ist ein wichtiges Einsatzprodukt zur Synthese von Alterungsschutzmitteln und Stabilisatoren in der Gummi- und Polymerindustrie (Kirk-Othmer, Encyclopedia of Chemical Technology, 4^{th} Edition, 1992, Vol 3, Seite 424-456; Ullmann's Encylopedia of Industrial Chemistry, 5^{th} Edition, Vol A3, 1985, Seite 91-111).

4-ADPA kann nach verschiedenen Methoden hergestellt werden. Eine Möglichkeit 4-ADPA herzustellen, ist die zweistufige Umsetzung von Anilin bzw. Anilinderivaten mit p-Nitrochlorbenzol in Gegenwart eines Säureakzeptors oder eines Neutralisierungsmittels, gegebenenfalls in Gegenwart eines Katalysators. Die Herstellung nach dieser Methode ist beispielsweise beschrieben in DE-A 3246151, DE-A 3501698, DE-A 185663, US-A 4670595, US-A 4187249, US-A 4683332, US-A 4187248 und J. Org. Chem. 42, 1786-1790 (1977). Die erste Stufe wird meist mit Kupfer-Katalysatoren, die zweite mit davon unterschiedlichen Metallkomponenten, z.B. Nickel, durchgeführt (siehe hierzu z.B. US-A 5840982). Umsetzungen von z.B. auch halogenierten Nitrobenzolen mit Aminen in Gegenwart von Palladium-Katalysatoren sind beschrieben in US-A 5576460 und EP-A 846676.

Die Nachteile der in der obigen Literatur beschriebenen Verfahren sind oftmals die unzureichenden Selektivitäten insbesondere bei der Bildung des Zwischenproduktes, wodurch sich Ausbeuteverlusten durch mehr oder weniger aufwendige Reinigungsschritte ergeben, bevor durch Hydrierung die 4-Aminodiphenylamine gebildet werden können.

Es war daher wünschenswert, ein Verfahren zur Herstellung von Aminodiphenylaminen zur Verfügung zu stellen, das von aromatischen Aminen ausgeht und durch Umsetzung mit entsprechenden Nitrohalogenbenzolen und anschließender Hydrierung des gebildeten Zwischenproduktes in guten Ausbeuten und hohen Reinheiten zu den gewünschten Aminodiphenylaminen führt.

Gegenstand der vorliegenden Erfindung ist daher ein Verfahren zur Herstellung von Aminodiphenylaminen durch Umsetzung von Nitrohalogenbenzolen mit aromatischen Aminen in Gegenwart einer Base und eines Palladium-Katalysators und anschließender Hydrierung des so erhaltenen Produkts mit Wasserstoff.

Als Nitrohalogenbenzole werden bevorzugt solche eingesetzt, in denen die Nitrogruppe in para-Stellung zum Halogenrest steht. Als Halogenreste kommen in Frage: Fluor, Chlor, Brom sowie Iod, bevorzugt Chlor und Brom. Selbstverständlich können die Nitrohalogenbenzole noch durch einen oder mehrere andere Reste substituiert sein, wie beispielsweise C₁-C₄-Alkylreste. Selbstverständlich kann die Stellung der Nitrogruppe zu den Halogenresten auch eine anderen als die para-Position sein, z.B. in 2- oder 3-Position.

Als Nitrohalogenbenzole sind beispielsweise zu nennen: 4-Nitro-2-methylchlorbenzol, 4-Nitro-3-methylchlorbenzol, 4-Nitrochlorbenzol, 3-Nitrochlorbenzol und 2-Nitrochlorbenzol. Besonders bevorzugt ist 4-Nitrochlorbenzol.

Als aromatische Amine können in das erfindungsgemäße Verfahren die für eine solche Reaktion bekannten aromatischen Amine eingesetzt werden, beispielsweise Anilin, o-Toluidin, m-Toluidin, p-Toluidin, 4-Ethylanilin, 4-Butylanilin, 4-Isopropylanilin, 3,5-Dimethylanilin oder 2,4-Dimethylanilin. Bevorzugt ist Anilin. Selbstverständlich können die aromatischen Amine auch in Form von Gemischen, insbesondere Isomeren-Gemischen, eingesetzt werden.

Bei dem erfindungsgemäßen Verfahren werden im allgemeinen pro Mol Nitrohalogenbenzol 1 bis 10 Mol, bevorzugt 1,5 bis 6 Mol, besonders bevorzugt 2 bis 4 Mol des aromatischen Amins eingesetzt.

Erfindungsgemäß können Palladium-Katalysatoren, z.B. Palladium-Phosphin-Komplexe, oder andere bekannte Palladiumverbindungen bzw. Komplexe eingesetzt werden.

Als Palladium-Phosphin-Komplexverbindungen sind solche geeignet, in denen das Palladium die Wertigkeit 0 oder II besitzt und als Phosphin-Liganden Verbindungen, wie Triphenylphosphin, Tri-o-toluylphosphin, Tricyclohexylphosphin, Tri-t-butylphosphin, Bisdiphenylphosphinethan, Bisdiphenylphosphinpropan, Bisdiphenylphosphinobutan, Bisdicyclohexylphosphinoethan, Bisdiphenylphosphino-ferrocen, 5,5'-Dichloro-6,6'-dimethoxy-biphenyl-2,2'-diyl-bis-diphenylphosphin, Bis-4,4'dibenzofuran-3,3'-yl-bisdiphenylphosphin, 1,1'-Bis-diphenylphosphino-diphenylether oder Bisdiphenylphosphinobinaphthyl, wobei die genannten Phenylreste durch Sulfonsäurereste substituiert sein können und/oder durch eine oder mehrere C₁-C₁₂-Alkylgruppen oder C₃-C₁₀-Cycloalkylgruppen. Ferner können auch polymergebundene Phosphine als Liganden dienen, z.B. tPP-Polymer (kommerziell erhältlich). Bevorzugt findet Triphenylphosphin als Ligand Verwendung.

Für das erfindungsgemäße Verfahren können aber auch andere als die erwähnten Palladium-Phosphin-Komplexverbindungen eingesetzt werden, wie beispielsweise Stickstoff- oder Sauerstoff-haltige Liganden, wie 1,10-Phenanthrolin, Diphenylethandiamin, [1,1']-Binaphthenyl-2,2'-diol (BINOL) sowie 1,1'-Binaphthenyl-2,2'-dithiol (BINAS), oder auch solche mit zwei oder mehreren verschiedenen Heteroatomen, wie O, N, S.

Als Palladium-Verbindungen, die als Katalysator dienen, sind beispielsweise folgende Verbindungsklassen zu nennen: Palladium-Halogenide, -acetate, -carbonate, -ketonate, -nitrate, -acetonate oder Palladacyclen, beispielsweise Pd₂dba₃, Pd(acac)₂, Pd(OAc)₂, PdCl₂, (CH₃CN)₂Pd(NO₂)Cl. Bevorzugt sind Pd₂dba₃, Pd(acac)₂, Pd(OAc)₂, PdCl_{2.} Ferner sind auch heterogene oder immobilisierte Palladiumkatalysatoren im erfindungsgemäßen Verfahren einsetzbar, d.h. solche die z.B. auf geeignete inerte Träger aufgebracht sind.

Bei den erfindungsgemäß einzusetzenden Palladium-Phosphin-Komplexen beträgt das molare Verhältnis des entsprechenden Liganden zu Palladium etwa 40:1 bis 1:1, bevorzugt 10:1 bis 2:1, besonders bevorzugt 8:1 bis 4:1.

Erfindungsgemäß werden die Palladium-Katalysatoren, wie Palladium-Phosphin-Komplexe und/oder die anderen einsetzbaren Komplexe oder Verbindungen, im allgemeinen in Mengen von 0,0001 Mol-% bis 10 Mol %, bevorzugt 0,001 Mol-% bis 5 Mol-%, bezogen auf die eingesetzten Nitrohalogenbenzole, eingesetzt.

Als Basen werden im erfindungsgemäßen Verfahren Alkali- und/oder Erdalkalimetallcarbonate, -alkoholate und/oder -hydroxide eingesetzt, wobei insbesondere Kalium- und/oder Natriumcarbonat, Cäsiumcarbonat, Natriummethanolat und Bariumhydroxid zu nennen sind. Bevorzugt werden Kalium- und/oder Natriumcarbonat eingesetzt. Die Basen können in unterstöchiometrischer Menge oder im Überschuss bis zur zehnfachen äquivalenten Menge bezüglich des Nitrohalogenbenzols eingesetzt werden. Besonders bevorzugt werden die Basen in der 0,3 bis 2 äquivalenten Menge, bezogen auf Nitrohalogenbenzol eingesetzt.

Für das erfindungsgemäße Verfahren ist es von Vorteil, wenn die eingesetzten Basen durch Mahlung und/oder Trocknung vorbehandelt werden.

Die Mahlung kann im erfindungsgemäßen Verfahren beispielsweise in handelsüblichen Mühlen erfolgen. Die Maßnahme des Mahlens bewirkt hierbei eine drastische Vergrößerung der spezifischen Oberfläche, die zu einer deutlichen Steigerung des Umsatzes führt. In vielen Fällen ist durch die Mahlung eine Vergrößerung der spezifischen Oberfläche um den Faktor 10 bis 20 zu beobachten.

Nach der Mahlung liegen die spezifischen Oberflächen der Basen bei ca. 0,1 bis 10 m²/g, bevorzugt 0,2 bis 1 m²/g (BET).

Aufgrund der ausgeprägten hygroskopischen Eigenschaften der im erfmdungsgemäßen Verfahren eingesetzten Basen, neigen diese zur mehr oder minder starken Aufnahme atmosphärischer Bestandteile, wie Wasser und Kohlendioxid. Ab einer Aufnahme von ca. 30 Gewichtsprozent an atmosphärischen Bestandteilen ist ein deutlicher Einfluss auf die zu erreichenden Umsätze feststellbar. Daher ist neben der Mahlung häufig auch eine Trocknung der Basen angezeigt.

Die Trocknung der Basen erfolgt dabei beispielsweise derart, dass unter vermindertem Druck von ca. 0,01 bis 100 mbar für mehrere Stunden auf Temperaturen von ca. 50 bis 200°C, bevorzugt 100 bis 160°C, erhitzt wird.

Die erste Stufe des erfindungsgemäßen Verfahrens kann bei Temperaturen im Bereich von 20 bis 250°C, bevorzugt bei Temperaturen von 120 bis 180°C, durchgeführt werden. Die Reaktionstemperaturen hängen dabei insbesondere von der Art der Ausgangsprodukte, des Katalysators und der eingesetzten Basen ab.

Das erfindungsgemäße Verfahren kann sowohl in Anwesenheit als auch in Abwesenheit eines geeigneten Lösungsmittels durchgeführt werden. Als Lösungsmittel kommen beispielsweise inerte, organische Kohlenwasserstoffe, wie Xylol und Toluol, in Frage. Weiterhin können die eingesetzten aromatischen Amine selbst als Lösungsmittel fungieren.

Bei dem erfindungsgemäßen Verfahren kann - wenn gewünscht - analog zu DE-A 26 33 811 und DE-A 32 46 151 das entstehende Reaktionswasser beispielsweise durch Zuhilfenahme eines geeigneten Schleppmittels durch Destillation entfernt werden.

Die Menge der eingesetzten Lösungsmittel kann leicht durch entsprechende Vorversuche bestimmt werden.

Die Durchführung des erfindungsgemäßen Verfahrens kann nach üblichen Methoden in kontinuierlicher oder diskontinuierlicher Weise erfolgen.

Beim erfindungsgemäßen Verfahren wird nach der Umsetzung der aromatischen Amine mit den Halogennitroaromaten das erhaltene Reaktionsprodukt mit Wasserstoff hydriert, wobei die Hydrierung in Gegenwart des bereits vorhandenen Palladiumkatalysators durchgeführt wird, gegebenenfalls unter Zusatz eines geeigneten, inerten Katalysatorträgers.

Selbstverständlich ist es auch möglich, die Hydrierung in Gegenwart zusätzlicher Hydrierkatalysatoren, wie solche auf Nickel-, Palladium- oder Platin-Basis, durchzuführen, gegebenenfalls unter Einsatz eines geeigneten Katalysatorträgers.

Geeignete Materialien für die Verwendung als Katalysatorträger sind alle technisch üblichen Katalysatorträger auf der Basis von Kohlenstoff, Elementoxiden, Elementcarbiden oder Elementsalzen in verschiedenen Anwendungsformen. Beispiele für kohlenstoffhaltige Träger sind Koks, Graphit, Ruß oder Aktivkohlen. Beispiele für die Elementoxid-Katalysatorträger sind SiO₂ (natürliche oder synthetische Kieselsäure, Quarz) Al₂O₃ (α, γ-Al₂O₃), Tonerden, natürliche oder synthetische Alumosilicate (Zeolithe), Schichtsilikate wie Bentonit und Montmorillonit, TiO₂ (Rutil, Anatas), ZrO₂, MgO oder ZnO. Beispiele für Elementcarbide und -salze sind SiC, AlPO₄, BaSO₄, CaCO₃. Grundsätzlich können sowohl synthetische Materialien als auch Träger aus natürlichen Quellen, wie Bimsstein, Kaolin, Bleicherden, Bauxite, Bentonite, Kieselgur, Asbest oder Zeolithe, verwendet werden.

Weitere brauchbare Träger für die erfindungsgemäß einsetzbaren Katalysatoren sind Element-Mischoxide und -Oxidhydrate von Elementen der Gruppen 2 bis 16 des Periodensystems sowie der Seltenerdmetalle (Atomnummern 58 bis 71), bevorzugt aus den Elementen Al, Si, Ti, Zr, Zn, Mg, Ca, Sn, Nb und Ce, die u.a. auf dem Weg über mechanische Vermischungen, gemeinsame Fällungen von Salzen oder über Cogele aus Salzen und/oder Alkoxiden hergestellt werden können, wie dies dem Fachmann bekannt ist.

Die Träger können sowohl im Sinne chemisch einheitlicher Reinsubstanzen als auch im Gemisch eingesetzt werden. Für die erfindungsgemäße Verwendung als Katalysatorträger eignen sich sowohl stückige als auch pulverförmige Materialien. Für den Fall der Anordnung des Träger-Katalysators als Festbett wird der Träger vorzugsweise als Formkörper, z.B. als Kugeln, Zylinder, Stäbchen, Hohlzylinder oder Ringe, eingesetzt. Wahlweise können Katalysatorträger weiter durch Extrudieren, Tablettieren, gegebenenfalls unter Zumischen weiterer Katalysatorträger oder Bindemittel, wie SiO₂ oder Al₂O₃, und Kalzinieren modifiziert werden. Die innere Oberfläche der Träger (BET-Oberfläche) liegt bei 1 bis 2000m²/g, bevorzugt bei 10 bis 1600 m²/g, ganz besonders bevorzugt bei 20 bis 1500 m²/g. Darstellung und Weiterverarbeitung sind dem Fachmann wohl bekannt und Stand der Technik.

Bevorzugt werden Aktivkohlen und Si-, Al-, Mg-, Zr- und Ti-haltige Materialien als Träger-materialien eingesetzt. Besonders bevorzugt ist Aktivkohle.

Die benannten Träger können auch mit Palladium mit einem Metallgehalt von 0,01 bis 50 Gew.%, bevorzugt 0,1 bis 10 Gew.%, bezogen auf das Gesamtgewicht des Katalysators, beladen sein.

Die benannten Trägermaterialien oder die mit Palladium beladenen Trägermaterialien können in Mengen von 0,01 bis 20 Gew.%, bezogen auf eingesetztes Halogennitrobenzol, eingesetzt werden, bevorzugt in Mengen von 0,01 bis 10 Gew.%. Bevorzugt ist die Verwendung von mit Palladium beladener Aktivkohle.

Selbstverständlich kann die Hydrierung auch mit anderen Reduktionsmethoden, wie sie dem Fachmann bekannt sind und z.B. in "Reductions in Organic Chemistry, Second Edition, ACS Monograph 188" aufgeführt sind, durchgeführt werden.

Die Temperaturen bei der Hydrierung betragen ca. 0 bis 200°C, insbesondere 40 bis 150°C; die Drücke (Wasserstoffdruck) liegen bei ca. 0,1 bis 150 bar, insbesondere 0,5 bis 70 bar, ganz besonders bevorzugt 1 bis 50 bar.

Nach dem erfindungsgemäßen Verfahren werden die entsprechenden 4-Aminodiphenylamine mit hohen Selektivitäten (>98 %) und in Ausbeuten bis zu 99 % erhalten.

### Beispiele

### Beispiel 1

In einem Mehrhals-Rundkolben werden 372.0 g (4.00 mol) Anilin, 0.25 g (0.00082 mol) Palladiumacetonylacetonat und 0.86 g (0.00328 mol) Triphenylphosphin unter Inertatmosphäre vorgelegt und 10 Minuten bei Raumtemperatur gerührt. Man gibt 157.6 g (1.00 mol) 4-Chlornitrobenzol zu und rührt für weitere 10 Minuten bei Raumtemperatur. Sodann werden 120.0 g (0.87 mol) gemahlenes Kaliumcarbonat und 40 ml Xylol zugegeben. Unter starkem Rühren wird für 45 min auf Rückfluss und unter Wasserabscheidung erhitzt. Gaschromatographische Kontrolle ergibt vollständigen Umsatz von para-Chlornitrobenzol.
Man lässt auf 85°C abkühlen und verdünnt mit 300 ml Wasser. Die organische Phase wird mit 1.0 g Pd/C (5 % Pd/C) 15 min bei 10 bar Wasserstoffdruck hydriert, wobei die Temperatur 110°C erreicht.
Nach Filtration und Destillation erhält man 182 g (99 % der Theorie) 4-Aminodiphenylamin.

### Beispiel 2

In einem Mehrhals-Rundkolben werden 372.0 g (4.00 mol) Anilin, 0.20 g (0.00066 mol) Palladiumacetonylacetonat und 0.69 g (0.00263 mol) Triphenylphosphin unter Inertatmosphäre vorgelegt und 10 Minuten bei Raumtemperatur gerührt. Man gibt 157.6 g (1.00 mol) 4-Chlornitrobenzol zu und rührt für weitere 10 Minuten bei Raumtemperatur. Sodann werden 120.0 g (0.87 mol) gemahlenes Kaliumcarbonat und 40 ml Xylol zugegeben. Unter starkem Rühren wird für 45 min auf Rückfluss und unter Wasserabscheidung erhitzt. Gaschromatographische Kontrolle ergibt vollständigen Umsatz von para-Chlornitrobenzol.
Man lässt auf 85°C abkühlen und verdünnt mit 300 ml Wasser. Die organische Phase wird mit 1.0 g Pd/C (3 % Pd/C) 11 min bei 10 bar Wasserstoffdruck hydriert, wobei die Temperatur 120°C erreicht.

Nach Filtration und Destillation erhält man 181 g (98 % der Theorie) 4-Aminodiphenylamin.

### Beispiel 3

In einem Mehrhals-Rundkolben werden 372.0 g (4.00 mol) Anilin, 0.25 g (0.00082 mol) Palladiumacetonylacetonat und 0.86 g (0.00328 mol) Triphenylphosphin unter Inertatmosphäre vorgelegt und 10 Minuten bei Raumtemperatur gerührt. Man gibt 157.6 g (1.00 mol) 4-Chlornitrobenzol zu und rührt für weitere 10 Minuten bei Raumtemperatur. Sodann werden 96.6 g (0.70 mol) gemahlenes Kaliumcarbonat und 50 ml Xylol zugegeben. Unter starkem Rühren wird für 45 min auf Rückfluss und unter Wasserabscheidung erhitzt. Gaschromatographische Kontrolle ergibt vollständigen Umsatz von para-Chlornitrobenzol.
Man lässt auf 85°C abkühlen und verdünnt mit 300 ml Wasser. Die organische Phase wird mit 1.0 g Pd/C (Beladung 5 % Pd/C) 14 min bei 10 bar Wasserstoffdruck hydriert, wobei die Temperatur 120°C erreicht.
Nach gaschromatographischer Untersuchung erhält man 99% 4-Aminodiphenylamin.

### Beispiel 4

In einem Mehrhals-Rundkolben werden 372.0 g (4.00 mol) Anilin, 0.22 g (0.00098 mol) Palladiumacetat und 1.04 g (0.00397 mol) Triphenylphosphin unter Inertatmosphäre vorgelegt und 10 Minuten bei Raumtemperatur gerührt. Man gibt 157.6 g (1.00 mol) 4-Chlornitrobenzol zu und rührt für weitere 10 Minuten bei Raumtemperatur. Sodann werden 96.6 g (0.70 mol) gemahlenes Kaliumcarbonat und 50 ml Xylol zugegeben. Unter starkem Rühren wird für 45 min auf Rückfluss und unter Wasserabscheidung erhitzt. Gaschromatographische Kontrolle ergibt vollständigen Umsatz von para-Chlornitrobenzol.
Die organische Phase wird 25 min bei 10 bar Wasserstoffdruck hydriert, wobei die Temperatur 140°C erreicht.

Nach gaschromatographischer Untersuchung erhält man 98 % 4-Aminodiphenylamin.

### Beispiel 5

In einem Mehrhals-Rundkolben werden 372.0 g (4.00 mol) Anilin, 0.30 g (0.00098 mol) Palladiumacetonylacetonat und 1.04 g (0.00397 mol) Triphenylphosphin unter Inertatmosphäre vorgelegt und 10 Minuten bei Raumtemperatur gerührt. Man gibt 157.6 g (1.00 mol) 4-Chlornitrobenzol zu und rührt für weitere 10 Minuten bei Raumtemperatur. Sodann werden 96.6 g (0.70 mol) gemahlenes Kaliumcarbonat und 50 ml Xylol zugegeben. Unter starkem Rühren wird für 45 min auf Rückfluss und unter Wasserabscheidung erhitzt. Gaschromatographische Kontrolle ergibt vollständigen Umsatz von para-Chlornitrobenzol.
Man lässt auf 85°C abkühlen und verdünnt mit 300 ml Wasser. Die organische Phase wird 34 min bei 10 bar Wasserstoffdruck hydriert, wobei die Temperatur 140°C erreicht.
Nach gaschromatographischer Untersuchung erhält man 99 % 4-Aminodiphenylamin.

### Beispiel 6

In einem Mehrhals-Rundkolben werden 372.0 g (4.00 mol) Anilin, 0.30 g (0.00098 mol) Palladiumacetonylacetonat und 1.04 g (0.00397 mol) Triphenylphosphin unter Inertatmosphäre vorgelegt und 10 Minuten bei Raumtemperatur gerührt. Man gibt 157.6 g (1.00 mol) 4-Chlornitrobenzol zu und rührt für weitere 10 Minuten bei Raumtemperatur. Sodann werden 96.6 g (0.70 mol) gemahlenes Kaliumcarbonat und 50 ml Xylol zugegeben. Unter starkem Rühren wird für 45 min auf Rückfluss und unter Wasserabscheidung erhitzt. Gaschromatographische Kontrolle ergibt vollständigen Umsatz von para-Chlornitrobenzol.

Man lässt auf 85°C abkühlen und verdünnt mit 300 ml Wasser. Die organische Phase wird nach Zugabe von 2.0 g Aktivkohle 24 min bei 10 bar Wasserstoffdruck hydriert, wobei die Temperatur 140°C erreicht.
Nach gaschromatographischer Untersuchung erhält man 99% 4-ADPA.

### Beispiel 7

Vorbehandlung der Basen:
Beispielsweise wird kommerziell verfügbares Kaliumkarbonat für ca. 5 Minuten in einer Küchen- bzw. Kugelmühle gemahlen. Das auf diese Weise behandelte Kaliumcarbonat der Firma Grüssing erfährt hierdurch eine Vergrößerung der spezifischen Oberfläche von 0,04 m²/g auf 0,52 m²/g und eine Primärkristallitgröße von 10 µm und kleiner. Anschließend wird das gemahlene Kaliumcarbonat für 5 Stunden bei einem Druck von 1 mbar und einer Temperatur von 150°C getrocknet. Bei Verwendung anderer Basen werden diese in analoger Weise vorbehandelt.

## Patentansprüche

1. Verfahren zur Herstellung von Aminodiphenylaminen, **dadurch gekennzeichnet, dass** man aromatische Amine mit Nitrohalogenbenzolen in Gegenwart eines Palladiumkatalysators und einer Base umsetzt und anschließend das so erhaltene Produkt mit Wasserstoff hydriert, wobei die Hydrierung in Gegenwart des bereits vorhandenen Palladiumkatalysators durchgeführt wird.

2. Verfahren nach Anspruch 1, **dadurch gekennzeichnet, dass** man als Nitrohalogenbenzole solche einsetzt, in denen die Nitrogruppe in para-Stellung zum Halogenrest steht.

3. Verfahren nach Anspruch 1, **dadurch gekennzeichnet, dass** man als aromatische Amine Anilin, o-Toluidin, m-Toluidin, p-Toluidin, 4-Ethylanilin, 4-Butylanilin, 4-Isopropylanilin, 3,5-Dimethylanilin oder 2,4-Dimethylanilin einsetzt.

4. Verfahren nach Anspruch 1, **dadurch gekennzeichnet, dass** man pro Mol Nitrohalogenbenzol 1 bis 10 mol an aromatischem Amin einsetzt.

5. Verfahren nach Anspruch 1, **dadurch gekennzeichnet, dass** man als Palladiumkatalysator Palladium-Phosphin-Komplexe einsetzt.

6. Verfahren nach Anspruch 5, **dadurch gekennzeichnet, dass** bei den Palladium-Phosphin-Komplexen das molare Verhältnis des Phosphins zu Palladium 40:1 bis 1:1 beträgt.

7. Verfahren nach Anspruch 1, **dadurch gekennzeichnet, dass** die Palladiumkatalysatoren in Mengen von 0,0001 Mol-% bis 10 Mol-%, bezogen auf die eingesetzten Nitrohalogenbenzole, verwendet werden.

8. Verfahren nach Anspruch 1, **dadurch gekennzeichnet, dass** die eingesetzten Basen durch Mahlung und/oder Trocknung vorbehandelt werden.

9. Verfahren nach Anspruch 1, **dadurch gekennzeichnet, dass** die spezifische Oberflächen der Basen nach Mahlung bei 0,1 bis 10 m²/g (BET) liegen.

## Claims

1. A process for producing aminodiphenylamines, **characterised in that** aromatic amines are reacted with nitrohalobenzenes in the presence of a palladium catalyst and a base and the product thus obtained is subsequently hydrogenated with hydrogen, wherein hydrogenation is performed in the presence of the palladium catalyst already present.

2. A process according to claim 1, **characterised in that** the nitrohalobenzenes used are preferably those in which the nitro group is in para-position relative to the halogen residue.

3. A process according to claim 1, **characterised in that** the aromatic amines used are aniline, o-toluidine, m-toluidine, p-toluidine, 4-ethylaniline, 4-butylaniline, 4-isopropylaniline, 3,5-dimethylaniline or 2,4-dimethylaniline.

4. A process according to claim 1, **characterised in that** 1 to 10 mol of aromatic amine is used per mol of nitrohalobenzene.

5. A process according to claim 1, **characterised in that** palladium/phosphine complexes are used as the palladium catalyst.

6. A process according to claim 5, **characterised in that**, in the palladium/phosphine complexes, the molar ratio of phosphine to palladium is 40:1 to 1:1.

7. A process according to claim 1, **characterised in that** the palladium catalysts are used in amounts of from 0.0001 mol% to 10 mol% relative to the nitrohalobenzenes used.

8. A process according to claim 1, **characterised in that** the bases used are pretreated by grinding and/or drying.

9. A process according to claim 1, **characterised in that** the specific surface areas of the bases after grinding amount to from 0.1 to 10 m²/g (BET).

## Revendications

1. Procédé de préparation d'aminodiphénylamines, **caractérisé en ce que** des amines aromatiques sont mises en réaction avec des nitrohalogénobenzènes en présence d'un catalyseur au palladium et d'une base et, ensuite, le produit ainsi obtenu est hydrogéné avec de l'hydrogène, l'hydrogénation se déroulant en présence du catalyseur au palladium déjà présent.

2. Procédé selon la revendication 1 **caractérisé en ce que** les nitrohalogénobenzènes utilisés sont ceux dans lesquels le radical nitro se trouve en position para par rapport au radical halogène.

3. Procédé selon la revendication 1, **caractérisé en ce que** les amines aromatiques utilisées sont l'aniline, la o-toluidine, la m-toluidine, la p-toluidine, la 4-éthylaniline, la 4-butylaniline, la 4-isopropylaniline, la 3,5-dimétylaniline ou la 2,4-diméthylaniline.

4. Procédé selon la revendication 1, **caractérisé en ce que** l'on utilise 1 à 10 moles d'amine aromatique par mole de nitrohalogénobenzène.

5. Procédé selon la revendication 1, **caractérisé en ce que** l'on utilise des complexes de palladium-phosphine comme catalyseur au palladium.

6. Procédé selon la revendication 5, **caractérisé en ce que** le rapport molaire de la phosphine au palladium est de 40:1 à 1:1 pour les complexes de palladium-phosphine.

7. Procédé selon la revendication 1, **caractérisé en ce que** les catalyseurs au palladium sont utilisés dans des quantités de 0,0001 % en mole à 10 % en mole par rapport aux nitrohalogénobenzènes utilisés.

8. Procédé selon la revendication 1, **caractérisé en ce que** les bases utilisées sont prétraitées par broyage et/ou séchage.

9. Procédé selon la revendication 1, **caractérisé en ce que** les superficies spécifiques des bases s'élèvent à 0,1 à 10 m²/g (BET) après broyage.
